# EUROPEAN PATENT SPECIFICATION

(11) **EP 2 481 336 B1**
(45) Date of publication and mention of the grant of the patent: **25.07.2018**
(21) Application number: 10818652.9
(22) Date of filing: 30.08.2010
(51) Int. Cl.: A61B 1/00, G02B 23/24, A61B 1/04

(54) **ENDOSCOPE**
ENDOSKOP
ENDOSCOPE

(30) Priority: 22.09.2009 US 244586 P; 16.12.2009 JP 2009285073
(43) Date of publication of application: 01.08.2012
(73) Proprietor: Olympus Corporation, Tokyo 192-8507 (JP)
(72) Inventor: OKAZAKI, Yoshiro, Hachioji-shi, Tokyo 192-8507 (JP); SUGAHARA, Michihiro, Hachioji-shi, Tokyo 192-8507 (JP)
(74) Representative: Schicker, Silvia
(86) International application number: PCT/JP2010/064674
(87) International publication number: WO 2011/036976

(56) References cited:
- EP-A1- 1 844 700
- EP-A1- 1 943 938
- WO-A1-2008/134457
- WO-A1-2008/134457
- WO-A1-2008/140117
- JP-A- 1 020 836
- JP-A- 62 035 318
- JP-A- 2003 144 378
- JP-A- 2004 097 391
- JP-A- 2007 054 333
- JP-U- 3 143 693
- US-A1- 2005 209 506

## Description

### {Technical Field}

The present invention relates to endoscopes.

### {Background Art}

There have hitherto been known techniques for temporarily immobilizing the heart by pressing a bar onto the surface of the heart and performing suction in order to stably perform an operation on the heart while suppressing the effect of pulsation (e.g. see PTL 1). Since the heart is temporarily immobilized, it becomes possible to attain a stable field of view and to perform an operation stably.

Furthermore, there are known techniques for inserting a flexible endoscope into the pericardial cavity between the heart and the pericardium and performing a procedure (e.g., see PTL 2).

EP 1 844 700 A1 discloses a device according to the preamble of claim 1.

US 2005/209506 A1 discloses several embodiments of a device to aid advancement of a colonoscope through a sigmoid colon which comprises an elongate body defining a colonoscope lumen for insertion of the colonoscope. The colonoscope can be protruded from and retracted into the elongate body.

WO 2008/140117 A1 discloses a stabilizer in which an endoscope is inserted and protrudes through an outlet port. A camera is mounted at the distal end of the endoscope. The stabilizer comprises a contact section.

WO 2008/134457 A1 discloses a manipulation assembly comprising a catheter sheath with a deployment catheter and an imaging hood that can be advanced and retracted relative to the catheter sheath. An imaging unit and an imaging lumen are also provided.

### {Citation List}

### {Patent Literature}

{PTL 1} PCT International Publication No. WO 97/10753
{PTL 2} U.S. Patent Application Publication No. 2004/0064138

### {Summary of Invention}

### {Technical Problem}

However, according to the techniques of PTL 1, since the heart is temporarily immobilized by securing the surface of the heart to a stationary object in the vicinity, such as an operating table or a rib, there is a disadvantage that a considerable burden is imposed on the patient.

Also, there is a problem with the techniques of PTL 2 in that, due to the effect of the pulsing heart, the distal end of an endoscope moves uncontrollably in the pericardial cavity, so that it is not possible to attain a stable field of view.

The present invention has been made in view of the situation described above, and it is an object thereof to provide an endoscope with which it is possible to attain a stable field of view without having to stop pulsation or the like of the heart or other tissues.

### {Solution to Problem}

To achieve the above-described object, the present invention provides the features defined in claim 1.

According to the present invention, by inserting the inserted portion into the body of a patient and securing at least the distal-end portion of the inserted portion to tissue inside the body with the securing means, regardless of the pulsation or the like of the tissue, it is possible to move the observation optical system provided at the inserted portion in synchronization with the pulsation of the tissue, thereby attaining a stable field of view. Furthermore, in this case, by adjusting the distance between the observation optical system and the tissue surface by the operation of the observation-distance adjusting means, it is possible to observe the tissue surface at a distance appropriate for the observation optical system. That is, according to the second aspect of the present invention, since tissue, such as the heart, is not immobilized to a stationary object in the vicinity, without having to stop the pulsation or the like of the tissue, it is possible to attain a stable field of view while alleviating the burden on the patient.

In the above invention, the securing means include an attachment unit that attaches to the tissue by using a negative pressure.

Accordingly, by supplying a negative pressure to the attachment unit so that the attachment unit attaches to the tissue, it is possible to readily secure at least the distal-end portion of the inserted portion to the tissue. Furthermore, by stopping the supply of the negative pressure, it is possible to readily release the inserted portion from the tissue.

In the above configuration, the attachment unit has an attachment surface for attachment to the tissue such that the angle of the attachment surface relative to the inserted portion is changeable.

When the attachment unit is attached to the tissue and the observation-distance adjusting means is operated, the inserted portion is moved in such a state that it is partially restrained by the attachment unit. Thus, by changing the angle of the attachment surface relative to the inserted portion, it is possible to adjust the distance between the observation optical system and the tissue surface while maintaining stable attachment without applying an undue force to the attachment surface or the inserted portion.

In the configuration wherein the angle of the attachment surface relative to the inserted portion is changeable, the attachment unit may include an attachment pad having the attachment surface and a joint that joins the attachment pad with the inserted portion so that the attachment pad is pivotable about an axis perpendicular to a lengthwise axis of the inserted portion.

Accordingly, by pivoting the attachment pad relative to the inserted portion via the joint with the attachment surface of the attachment pad attached to the tissue, the angle of the attachment surface relative to the inserted portion is changed. Thus, it is possible to adjust the distance between the observation optical system and the tissue surface while maintaining stable attachment without applying an undue force to the attachment surface or the inserted portion.

In the configuration wherein the attachment unit includes the attachment pad and the joint, the joint may be a flexible tubular member that conveys a negative pressure to the attachment pad.

Accordingly, by curving the tubular member with the attachment surface of the attachment pad attached to the tissue by the negative pressure conveyed by the tubular member, it is possible to change the angle of the attachment surface relative to the inserted portion.

In the above configuration, the attachment unit comprises a plurality of suction holes provided on a side face of the inserted portion.

Accordingly, by supplying a negative pressure to the suction holes so that the attachment unit attaches to the tissue, it is possible to secure the side face of the inserted portion to the tissue.

In the above configuration, a plurality of the attachment units may be provided at intervals along a lengthwise direction, and the observation optical system may be provided between a pair of the attachment units.

Accordingly, it is possible to secure the inserted portion to the tissue on both sides of the observation optical system, so that it is possible to move the observation optical system in synchronization with the tissue even when the tissue pulses, thereby attaining a stable field of view.

### {Advantageous Effects of Invention}

According to the present invention, an advantage is afforded in that it is possible to attain a stable field of view without having to stop the pulsation or the like of the heart or other tissues.

### {Brief Description of Drawings}

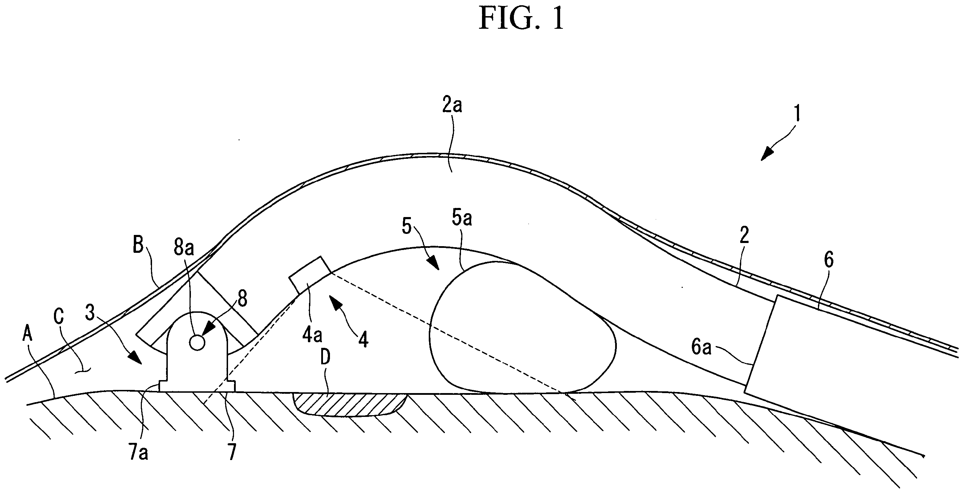

{Fig. 1}
   Fig. 1 is a front view of a distal-end portion of an inserted portion of an endoscope according to a first embodiment of the present invention.
{Fig. 2}
   Fig. 2 is a schematic longitudinal sectional view showing, in a further enlarged fashion, the distal end of the inserted portion of the endoscope shown in Fig. 1.
{Fig. 3A}
   Fig. 3A is an illustration for explaining an insertion operation of the endoscope shown in Fig. 1.
{Fig. 3B}
   Fig. 3B is an illustration for explaining an insertion operation of the endoscope shown in Fig. 1.
{Fig. 3C}
   Fig. 3C is an illustration for explaining an insertion operation of the endoscope shown in Fig. 1.
{Fig. 3D}
   Fig. 3D is an illustration for explaining an insertion operation of the endoscope shown in Fig. 1.
{Fig. 4}
   Fig. 4 is a front view showing an example of an instrument that is projected from the exit of a channel provided in the endoscope shown in Fig. 1.
{Fig. 5}
   Fig. 5 is a front view showing a modification of the placement of a balloon, an observation window, and the channel of the endoscope shown in Fig. 4.
{Fig. 6A}
   Fig. 6A is a longitudinal sectional view showing a modification of an attachment pad of the endoscope shown in Fig. 1.
{Fig. 6B}
   Fig. 6B is an illustration of the modification of the attachment pad of the endoscope shown in Fig. 1, as viewed from the side of an attachment surface.
{Fig. 7A}
   Fig. 7A is a front view for explaining a modification of the endoscope shown in Fig. 1, in which two attachment pads are provided.
{Fig. 7B}
   Fig. 7B is a front view for explaining a modification of the endoscope shown in Fig. 1, in which the viewing direction is straight ahead or oblique.
{Fig. 8}
   Fig. 8 is a partial longitudinal sectional view for explaining a moving mechanism of one of the attachment pads of the endoscope shown in Fig. 7A;
{Fig. 9A}
   Fig. 9A is a longitudinal sectional view for explaining an operation of a modification of the endoscope shown in Fig. 1, in which a hook member is provided as securing means and which does not form a part of the invention;
{Fig. 9B}
   Fig. 9B is a longitudinal sectional view for explaining an operation of a modification of the endoscope shown in Fig. 1, in which a hook member is provided as securing means and which does not form a part of the invention;
{Fig. 9C}
   Fig. 9C is a longitudinal sectional view for explaining an operation of a modification of the endoscope shown in Fig. 1, in which a hook member is provided as securing means and which does not form a part of the invention;
{Fig. 10}
   Fig. 10 is an illustration for explaining a modification of the endoscope shown in Fig. 1, in which a balloon member is provided as securing means and which does not form a part of the invention;
{Fig. 11}
   Fig. 11 is an illustration for explaining a modification of the endoscope shown in Fig. 1, in which forceps are provided as securing means and which does not form a part of the invention;
{Fig. 12}
   Fig. 12 is a front view for explaining a modification of the endoscope shown in Fig. 1, in which bellows are used as a joint that joints an attachment pad with the inserted portion;
{Fig. 13A}
   Fig. 13A is a partial perspective view for explaining a modification of the endoscope shown in Fig. 1, in which a strip-shaped elastic member is provided as observation-distance adjusting means;
{Fig. 13B}
   Fig. 13B is a partial perspective view for explaining a modification of the endoscope shown in Fig. 1, in which a strip-shaped elastic member is provided as observation-distance adjusting means;
{Fig. 14A}
   Fig. 14A is a perspective view for explaining an operation of the elastic member shown in Figs. 13A and 13B;
{Fig. 14B}
   Fig. 14B is a perspective view for explaining an operation of the elastic member shown in Figs. 13A and 13B;
{Fig. 15A}
   Fig. 15A is a perspective view showing a bundle of wires that serves as the elastic member shown in Figs. 14A and 14B;
{Fig. 15B}
   Fig. 15B is a perspective view showing a bundle of wires that serves as the elastic member shown in Figs. 14A and 14B;
{Fig. 16}
   Fig. 16 is a front view showing a modification of the endoscope shown in Fig. 1, in which a link mechanism is provided as observation-distance adjusting means;
{Fig. 17A}

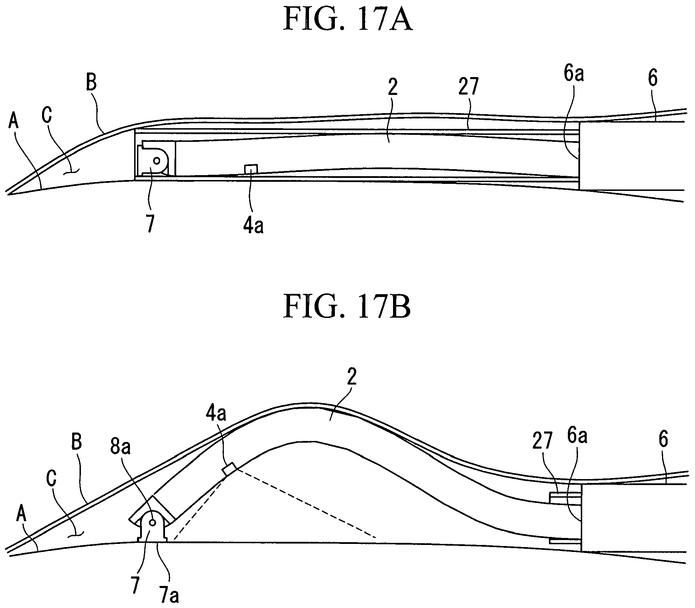

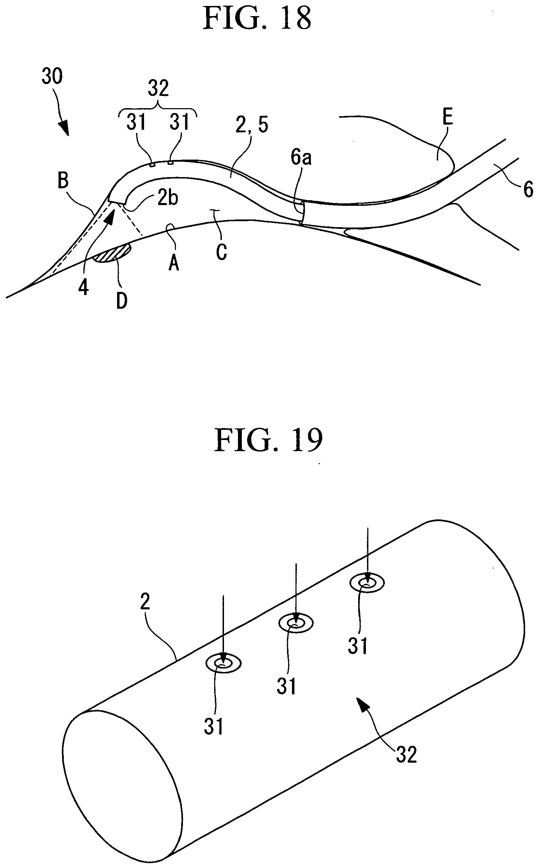

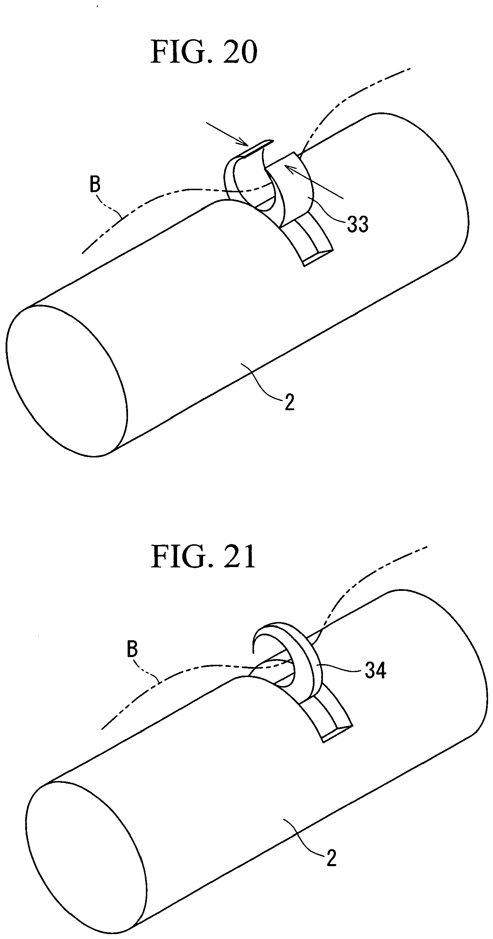

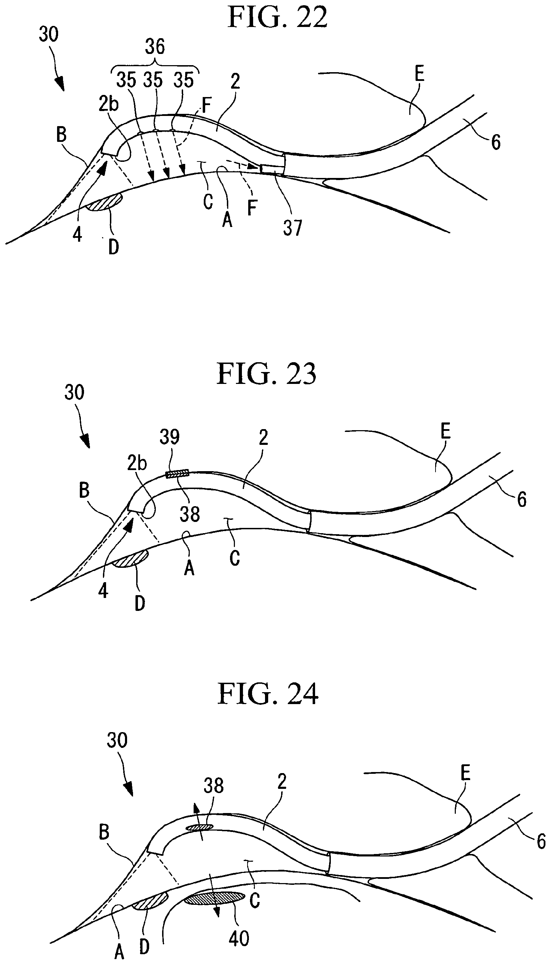

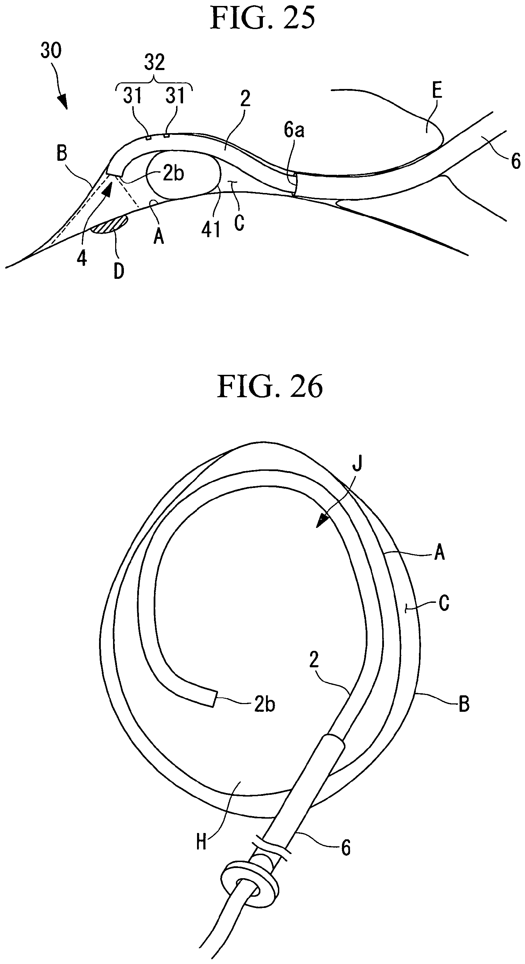

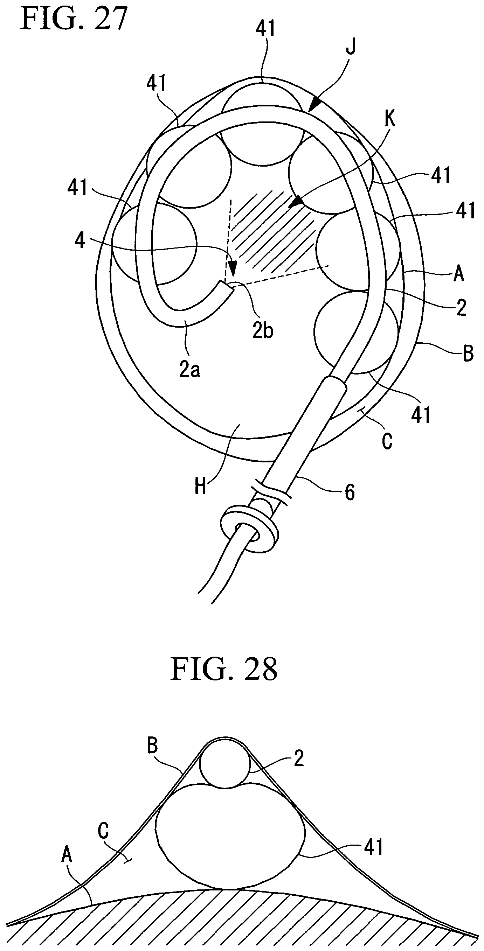

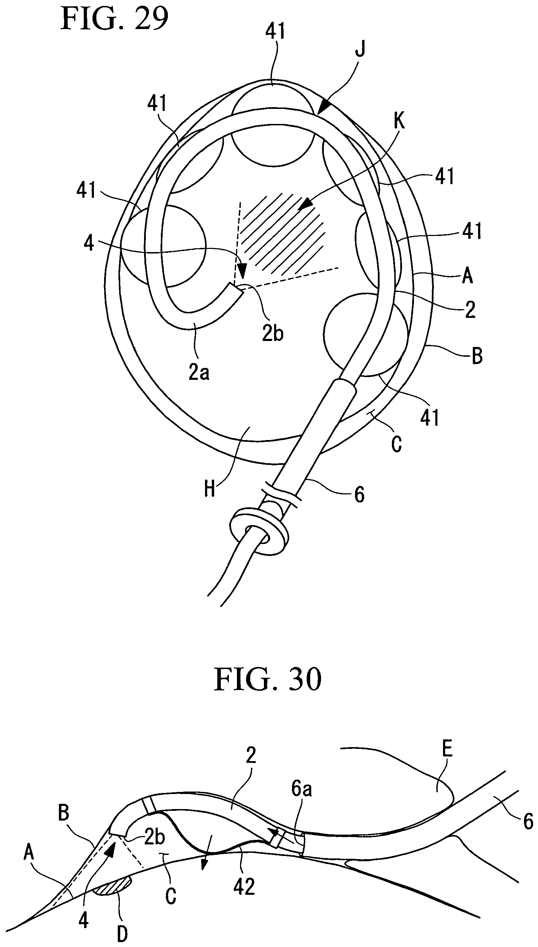

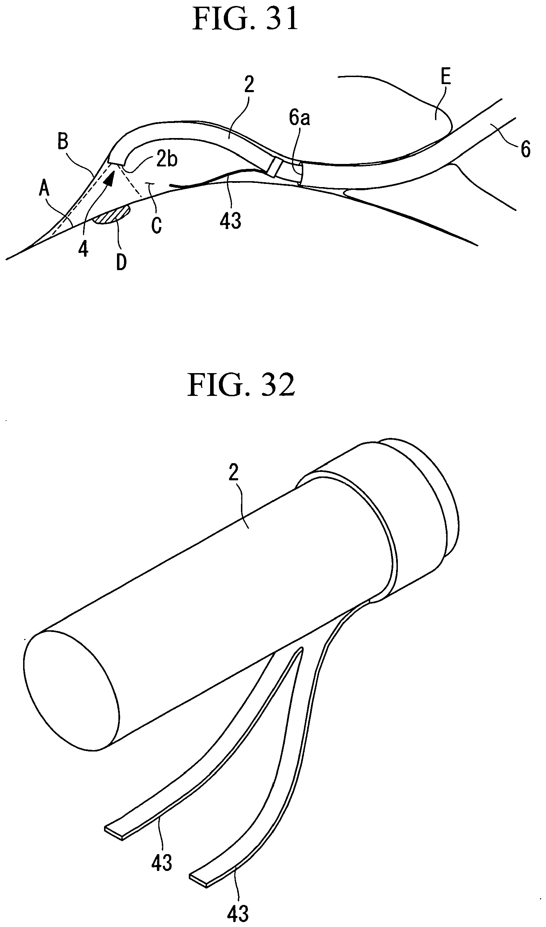

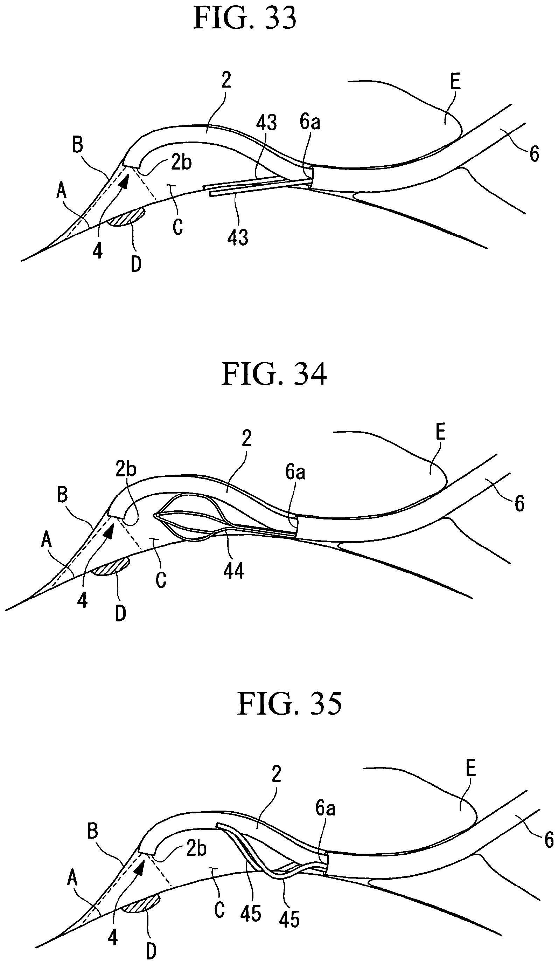

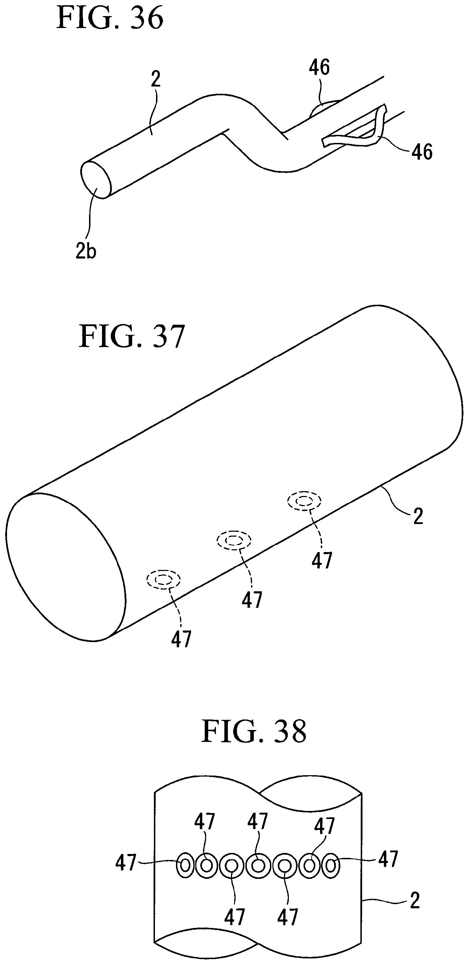

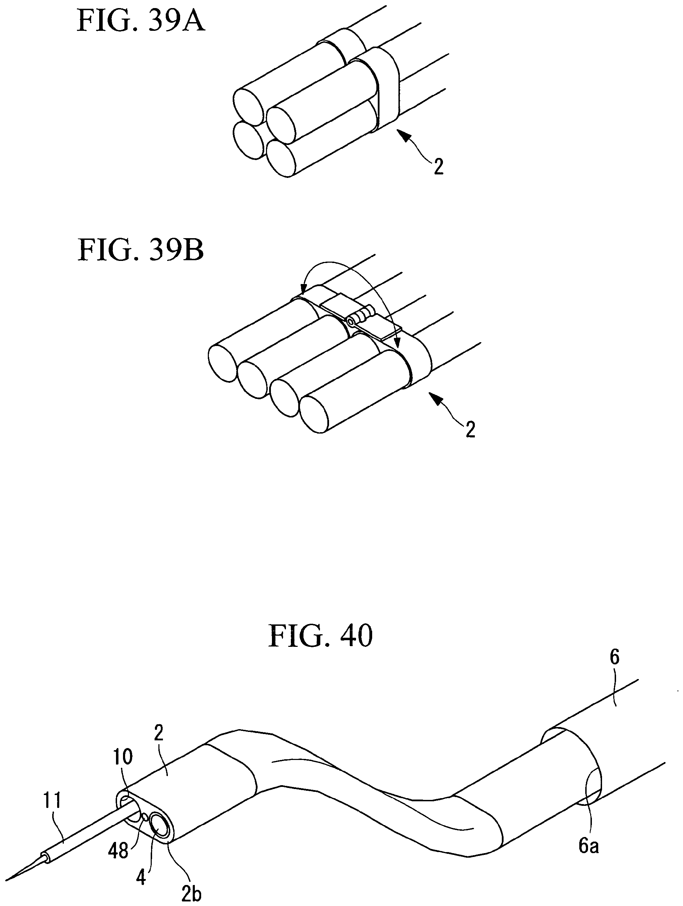

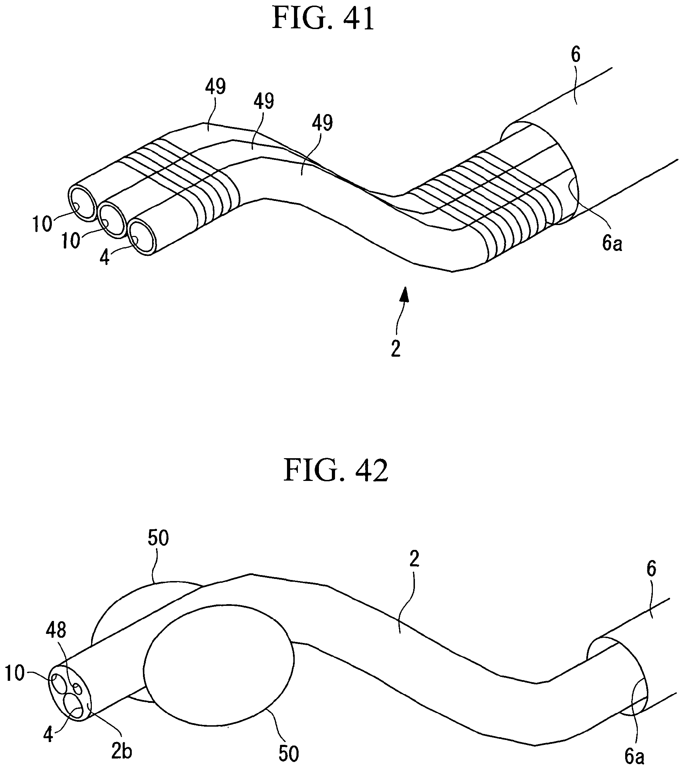

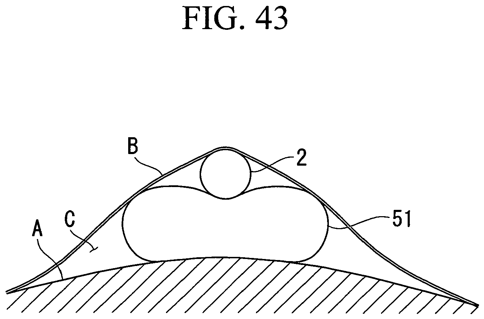

   Fig. 17A is a partially cutaway front view showing a modification of the endoscope shown in Fig. 1, in which an inserted portion having a pretrained shape is provided as observation-distance adjusting means;
{Fig. 17B}
   Fig. 17A is a partially cutaway front view showing a modification of the endoscope shown in Fig. 1, in which an inserted portion having a pretrained shape is provided as observation-distance adjusting means;
{Fig. 18}
   Fig. 18 is a front view of a distal-end portion of an inserted portion of an endoscope according to an example useful for understanding the invention;
{Fig. 19}
   Fig. 19 is a partial perspective view of the inserted portion shown in Fig. 18, showing securing means implemented by suction holes provided at the inserted portion;
{Fig. 20}
   Fig. 20 is a partial perspective view of an inserted portion, showing a modification of the securing means shown in Fig. 19;
{Fig. 21}
   Fig. 21 is a partial perspective view of an inserted portion, showing another modification of the securing means shown in Fig. 19;
{Fig. 22}
   Fig. 22 is a front view of a distal-end portion of an inserted portion, showing a modification including securing means different from that of the endoscope shown in Fig. 18;
{Fig. 23}
   Fig. 23 is a front view of a distal-end portion of an inserted portion, showing a modification including another securing means different from that of the endoscope shown in Fig. 18;
{Fig. 24}
   Fig. 24 is a front view of a distal-end portion of an inserted portion, showing a modification including another securing means different from that of the endoscope shown in Fig. 18;
{Fig. 25}
   Fig. 25 is a front view of a distal-end portion of an inserted portion, showing a modification including observation-distance adjusting means different from that of the endoscope shown in Fig. 18;
{Fig. 26}
   Fig. 26 is a plan view of a distal-end portion of an inserted portion, showing a modification including another observation-distance adjusting means different from that of the endoscope shown in Fig. 18;
{Fig. 27}
   Fig. 27 is a plan view of the distal-end portion of the inserted portion, showing a state where all the balloons constituting the observation-distance adjusting means of the endoscope shown in Fig. 26 are inflated;
{Fig. 28}
   Fig. 28 is a cross-sectional view of the balloons and the inserted portion of the endoscope shown in Fig. 27;
{Fig. 29}
   Fig. 29 is a plan view of the distal-end portion of the inserted portion, showing a state where some of the balloons constituting the observation-distance adjusting means of the endoscope shown in Fig. 26 are inflated;
{Fig. 30}
   Fig. 30 is a front view of a distal-end portion of an inserted portion, showing a modification including another observation-distance adjusting means different from that of the endoscope shown in Fig. 18;
{Fig. 31}
   Fig. 31 is a front view of a distal-end portion of an inserted portion, showing a modification including another observation-distance adjusting means different from that of the endoscope shown in Fig. 18;
{Fig. 32}
   Fig. 32 is a partial perspective view of an inserted portion, showing a modification of the observation-distance adjusting means shown in Fig. 31;
{Fig. 33}
   Fig. 33 is a front view of a distal-end portion of an inserted portion, showing a modification including another observation-distance adjusting means different from that of the endoscope shown in Fig. 18;
{Fig. 34}
   Fig. 34 is a front view of a distal-end portion of an inserted portion, showing a modification including another observation-distance adjusting means different from that of the endoscope shown in Fig. 18;
{Fig. 35}
   Fig. 35 is a front view of a distal-end portion of an inserted portion, showing a modification including another observation-distance adjusting means different from that of the endoscope shown in Fig. 18;
{Fig. 36}
   Fig. 36 is a partial perspective view showing an inserted portion of an endoscope, provided with means for stabilizing the inserted portion relative to the pulsation of the heart;
{Fig. 37}
   Fig. 37 is a partial perspective view showing an inserted portion of an endoscope, provided with another means for stabilizing the inserted portion relative to the pulsation of the heart;
{Fig. 38}
   Fig. 38 is a partial perspective view showing an inserted portion of an endoscope, provided with another means for stabilizing the inserted portion relative to the pulsation of the heart;
{Fig. 39A}
   Fig. 39A is a partial perspective view showing an inserted portion of an endoscope, provided with another means for stabilizing the inserted portion relative to the pulsation of the heart, showing a state where the inserted portion is tightly bundled;
{Fig. 39B}
   Fig. 39B is a partial perspective view showing an inserted portion of an endoscope, provided with another means for stabilizing the inserted portion relative to the pulsation of the heart, showing a state where the inserted portion is flatly expanded;
{Fig. 40}
   Fig. 40 is a partial perspective view showing an inserted portion of an endoscope, provided with another means for stabilizing the inserted portion relative to the pulsation of the heart;
{Fig. 41}
   Fig. 41 is a partial perspective view showing an inserted portion of an endoscope, provided with another means for stabilizing the inserted portion relative to the pulsation of the heart;
{Fig. 42}
   Fig. 42 is a partial perspective view showing an inserted portion of an endoscope, provided with another means for stabilizing the inserted portion relative to the pulsation of the heart; and
{Fig. 43}
   Fig. 43 is a cross-sectional view showing an inserted portion of an endoscope, provided with another means for stabilizing the inserted portion relative to the pulsation of the heart.

### {Description of Embodiments}

An endoscope 1 according to a first embodiment of the present invention will be described below with reference to the drawings.

As shown in Fig. 1, the endoscope 1 according to this embodiment includes a long, thin, flexible inserted portion 2 that is inserted into a body, securing means 3 that secures the distal end of the inserted portion 2 to tissue (e.g., the heart A) inside the body, an observation optical system 4 that is provided on the side face of the inserted portion 2 and that acquires images in outward radial directions, and observation-distance adjusting means 5 that adjusts the distance between the observation optical system 4 and the surface of the heart A.

Fig. 1 is an illustration showing the state of the distal end of the inserted portion 2 of the endoscope 1 inserted into the pericardial cavity C between the heart A and the pericardium B by a guide sheath 6.

As shown in Fig. 1, the inserted portion 2 has a curving portion 2a at a distal-end portion thereof, which can be curved in order to orient the distal-end face in an arbitrary direction.

The inserted portion 2 has an outer dimension smaller than the inner diameter of the guide sheath 6. When the inserted portion 2 is inserted into the pericardial cavity C, the inserted portion 2 is accommodated inside the guide sheath 6 such that it can be projected and retracted through a distal-end opening 6a of the guide sheath 6.

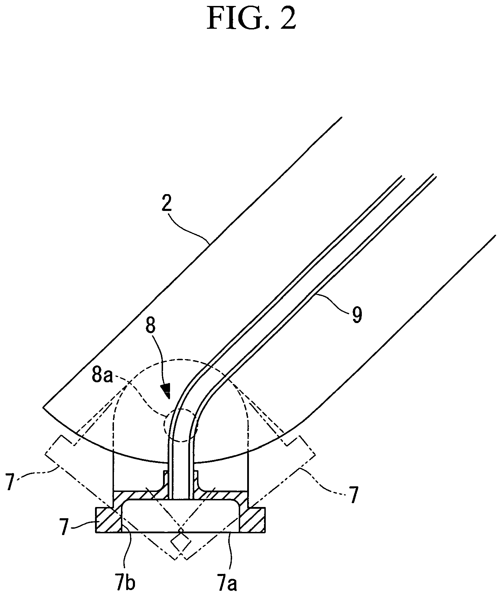

As shown in Fig. 2, the securing means 3 includes an attachment pad 7 provided at the distal end of the inserted portion 2, a joint 8 that joins the attachment pad 7 pivotally with the distal end of the inserted portion 2, and a duct 9 for supplying a negative pressure to an attachment surface 7a of the attachment pad 7. For example, the attachment pad 7 is composed of an elastic material, such as polyurethane rubber or silicone rubber. The attachment pad 7 has a flat attachment surface 7a having a suction hole 7b that can be opened in one direction.

The joint 8 includes a shaft 8a that is disposed along a radial direction of the inserted portion 2, and the joint 8 joins the attachment pad 7 with the inserted portion 2 pivotally about the shaft 8a. Thus, it is possible to change the angle of the attachment surface 7a in one direction with respect to the lengthwise axis of the inserted portion 2. Furthermore, by supplying a negative pressure to the attachment surface 7a of the attachment pad 7 via the duct 9, it is possible to attach the attachment pad 7 to the heart A disposed so as to close the suction hole 7b.

The observation optical system 4 has an observation window 4a provided on the side face of the inserted portion 2 at a position slightly toward the proximal end from the distal end. Inside the inserted portion 2, there are provided an objective lens (not shown) that collects light entering from radially outward of the inserted portion 2 through the observation window 4a and an image capturing element (not shown), such as a CCD, for forming images by using the light collected by the objective lens.

The observation-distance adjusting means 5 includes a balloon 5a disposed on the side face of the inserted portion 2 and a pipe (not shown) for supplying compressed air to the balloon 5a. The balloon 5a is secured at a circumferential position substantially corresponding to the observation window 4a of the inserted portion 2 and is connected to the flue provided inside the inserted portion 2. When the inserted portion 2 is inserted into the body, at which time the inserted portion 2 is accommodated inside the guide sheath 6, the balloon 5a is deflated and is disposed over the circumferential surface of the inserted portion 2. When the inserted portion 2 is projected through the distal-end opening 6a of the guide sheath 6 as shown in Fig. 1, the balloon 5a is inflated by compressed air supplied through the flue.

By inflating the balloon 5a, the surface of the heart A is pressed by the balloon 5a, so that the inserted portion 2 is moved away from the surface of the heart A. This makes it possible to adjust the distance between the observation window 4a provided on the side face of the inserted portion 2 and the surface of the heart A. The observation optical system 4 has a sufficient depth of field to enable focusing within a predetermined distance range from the observation window 4a. Thus, the balloon 5a is configured so that it is possible to adjust the distance between the observation window 4a and the surface of the heart A within a distance range covering the depth of field. The balloon 5a is composed of an elastic material, such as polyurethane rubber or silicone rubber.

The operation of the thus-configured endoscope 1 according to this embodiment will be described below.

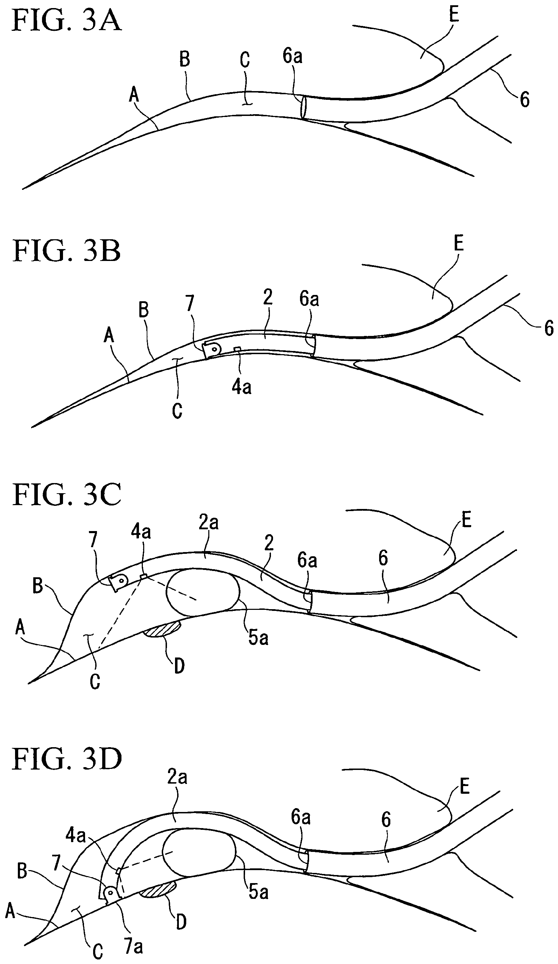

In order to observe tissue inside a body by using the endoscope 1 according to this embodiment, for example, a necrosed region D or the like existing on the outer surface of the heart A, as shown in Fig. 3A, the guide sheath 6 accommodating the inserted portion 2 and the attachment pad 7 is inserted into the pericardial cavity C from the bottom of the xiphoid process E through the pericardium B while observing the guide sheath 6 under x-ray radiography. In this state, as shown in Fig. 3B, the inserted portion 2 of the endoscope 1 inside the guide sheath 6 is pushed out from the distal-end opening 6a of the guide sheath 6.

In this state, as shown in Fig. 3C, the balloon 5a disposed between the inserted portion 2 and the surface of the heart A is inflated, whereby the inserted portion 2 is freed from the surface of the heart A. Accordingly, a distance is provided between the observation window 4a provided on the side face of the inserted portion 2 and the surface of the heart A, whereby an appropriate observation distance is formed. Then, the observation optical system 4 is operated to acquire an image of the surface of the heart A, and an affected area existing on the surface of the heart A, such as the necrosed region D, is observed.

Then, as shown in Fig. 3D, the curving portion 2a provided at the inserted portion 2 is operated so that the attachment surface 7a of the attachment pad 7 provided at the distal end of the inserted portion 2 approaches the surface of the heart A. Then, a negative pressure is supplied to the attachment pad 7 so that the attachment surface 7a is attached to the surface of the heart A. Thus, the inserted portion 2 is secured to the heart A, so that it is possible to move the inserted portion 2 in synchronization with pulsation of the heart A. Accordingly, it becomes possible to acquire substantially still images of an affected area, such as the necrosed region D, regardless of the pulsation of the heart A. That is, an advantage is afforded in that it is possible to perform observation stably without having to stop pulsation of the heart A.

In this case, with the endoscope 1 according to this embodiment, since the attachment pad 7 is pivotally joined with the inserted portion 2 by the joint 8, it is possible to readily change the angle of the inserted portion 2 relative to the attachment pad 7 attached to the surface of the heart A. Thus, application of undue forces to the attachment pad 7 or the inserted portion 2 is prevented, so that it is possible to keep the attachment surface 7a of the attachment pad 7 properly attached to the surface of the heart A.

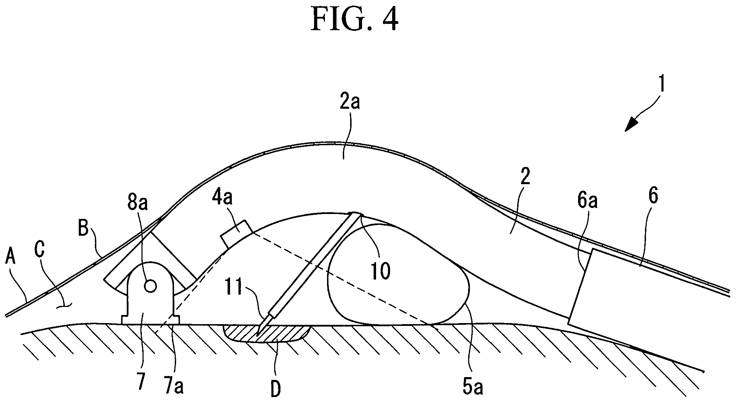

In the endoscope 1 according to this embodiment, as shown in Fig. 4, a channel for guiding an instrument may be provided. The channel is provided from the proximal end of the inserted portion 2 along the lengthwise direction and extends to an exit 10 provided between the balloon 5a and the observation window 4a. Thus, an instrument (e.g., a needle) 11 inserted from the proximal end of the inserted portion 2 is projected from the exit 10, which is provided on the distal-end side of the inserted portion 2 relative to the balloon 5a. The exit 10 is preferably provided at a circumferential position substantially corresponding to the observation window 4a. Furthermore, the exit 10 is preferably provided so that the distal end of the instrument 11 is projected within the field of view of the observation optical system 4. Accordingly, it is possible to manipulate the instrument 11 projected from the exit 10 while viewing it with the observation optical system 4.

In this case, according to this embodiment, the attachment pad 7 is attached to the surface of the heart A to secure the inserted portion 2 to the heart A. Thus, it is possible to immobilize the exit 10 of the channel relative to an affected area, such as the necrosed region D. Accordingly, an advantage is afforded in that it is possible to perform a procedure stably regardless of pulsation of the heart A.

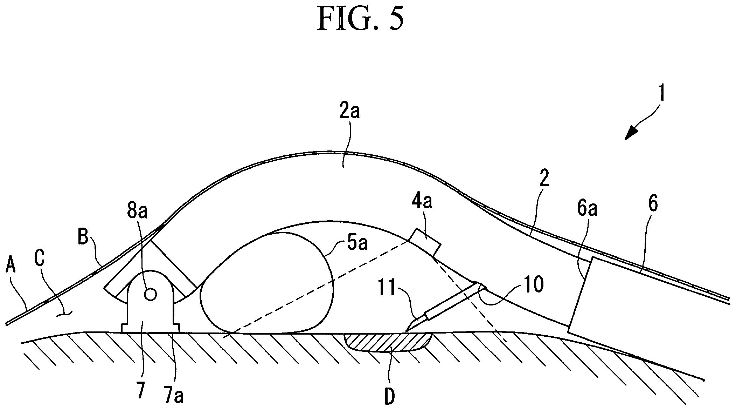

Although the exit 10 of the channel is provided between the balloon 5a and the observation window 4a in this embodiment, alternatively, as shown in Fig. 5, when the balloon 5a is provided on the distal-end side relative to the observation window 4a, the exit 10 of the channel may be provided on the proximal-end side of the inserted portion 2 relative to the observation window 4a.

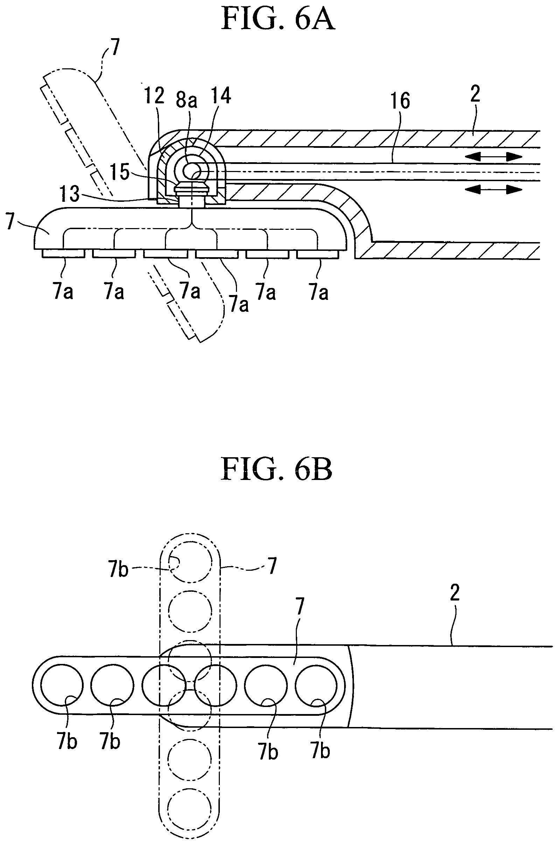

Furthermore, although an example has been described in which the attachment pad 7 has the single suction hole 7b and is attached so as to pivot only in one direction, alternatively, as shown in Figs. 6A and 6B, the attachment pad 7 may have a plurality of suction holes 7b and be attached so as to pivot about two mutually perpendicular axes.

More specifically, the attachment pad 7 is attached to a pivot member 12 attached at the distal end of the inserted portion 2 so as to pivot about the shaft 8a disposed perpendicularly to the lengthwise axis such that the attachment pad 7 is pivotable about an axis 13 that is perpendicular to the lengthwise axis of the inserted portion 2 and to the shaft 8a. Thus, by pivoting the pivot member 12 relative to the inserted portion 2 and rotating the attachment pad 7 relative to the pivot member 12, it is possible to pivot the attachment pad 7 about the mutually perpendicular two axes.

The attachment pad 7 is rotatable by a rotary driving mechanism configured of a driving gear 14 provided at the shaft 8a, a driven gear 15 provided at the attachment pad 7 and engaged with the driving gear 14, and a wire 16 wound on the shaft 8a. That is, regardless of the pivot angle of the pivot member 12, by pushing and pulling the wire 16 at the proximal end of the inserted portion 2, the shaft 8a is rotated to rotate the driving gear 14, thereby rotating the driven gear 15 engaged with the driving gear 14, whereby the attachment pad 7 to which the driven gear 15 is fixed can be rotated about the axis 13.

In this case, as indicated by solid lines in Figs. 6A and 6B, the inserted portion 2 is inserted into the body with the attachment pad 7 oriented along the lengthwise axis of the inserted portion 2, and inside the body, the attachment pad 7 is rotated about the axis 13 so that it is oriented as indicated by a chain line in Fig. 6B. Thus, the inserted portion 2 remains attached to the surface of the heart A against a torsion moment about the lengthwise axis applied to the inserted portion 2. Accordingly, it is possible to support the inserted portion 2 more stably so that the inserted portion 2 is secured relative to the heart A regardless of pulsation of the heart A.

In this case, preferably, negative pressures are supplied independently to the plurality of suction holes 7b provided on the attachment pad 7. That is, since the surface of the tissue of the heart A is not flat, in some cases, it is not possible to simultaneously attach all the suction holes 7b. Even in such cases, by supplying negative pressures independently, the plurality of suction holes 7a can be attached individually.

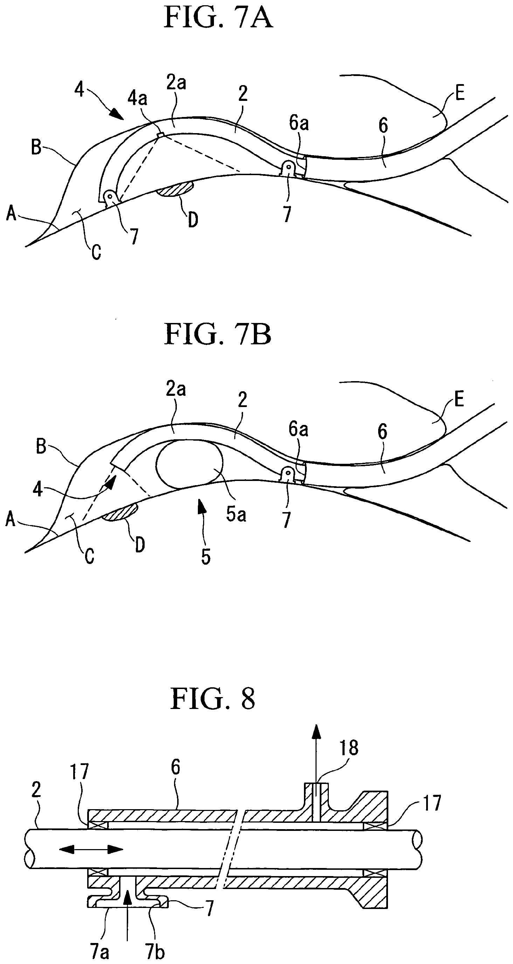

Furthermore, although the attachment pad 7 is provided only at the distal end of the inserted portion 2 in this embodiment, alternatively, as shown in Fig. 7A, attachment pads 7 may be provided pivotally at two or more positions at the distal end and a midway position of the inserted portion 2 on either side of the curving portion 2a along the lengthwise direction. Accordingly, by attaching the attachment pads 7 to the surface of the heart A at two or more positions, it is possible to secure the inserted portion 2 to the heart A more reliably. Furthermore, by operating and curving the curving portion 2a with the attachment pads 7 attached at two or more positions on either side of the curving portion 2a, as shown in Fig. 7A, it is possible to configure observation-distance adjusting means for adjusting the distance between the observation window 4a and an affected area, such as the necrosed region D.

Alternatively, as shown in Fig. 7B, the observation optical system 4 may be provided at the distal end of the endoscope 1 (the viewing direction may be oblique or straight ahead). In this case, the attachment pad 7 should be disposed on the proximal-end side relative to the balloon 5a constituting the observation-distance adjusting means 5. Accordingly, it is possible to curve the distal end of the inserted portion 2 by using the curving portion 2a, thereby readily changing the viewing direction.

Alternatively, as shown in Fig. 8, the attachment pad 7 provided at a midway position of the inserted portion 2 may be fixed at the distal end of the guide sheath 6 extending outside the body of the patient. In Fig. 8, the guide sheath 6 is sealed by sealing members 17 at the distal end and the proximal end, forming a cylindrical space with the inserted portion 2, and supports the inserted portion 2 so that the inserted portion 2 is movable along the lengthwise direction. Furthermore, at the proximal end of the guide sheath 6, a suction hole 18 connected to suction means (not shown) is provided at a position outside the body of the patient, and a negative pressure is supplied to the attachment surface 7a of the attachment pad 7 through the cylindrical space between the guide sheath 6 and the inserted portion 2.

Thus, by moving the inserted portion 2 relative to the guide sheath 6 along the lengthwise direction, it is possible to arbitrarily adjust the gap between the attachment pad 7 provided at the distal end of the inserted portion 2 and the attachment pad 7 provided at the distal end of the guide sheath 6. Accordingly, it is possible to adjust the gap in accordance with the size of the tissue to be attached, such as the heart A, thereby achieving an appropriately attached state.

Alternatively, instead of the attachment pad 7 provided at the midway position of the inserted portion 2, a suction hole 7b may be provided on the side face of the inserted portion 2.

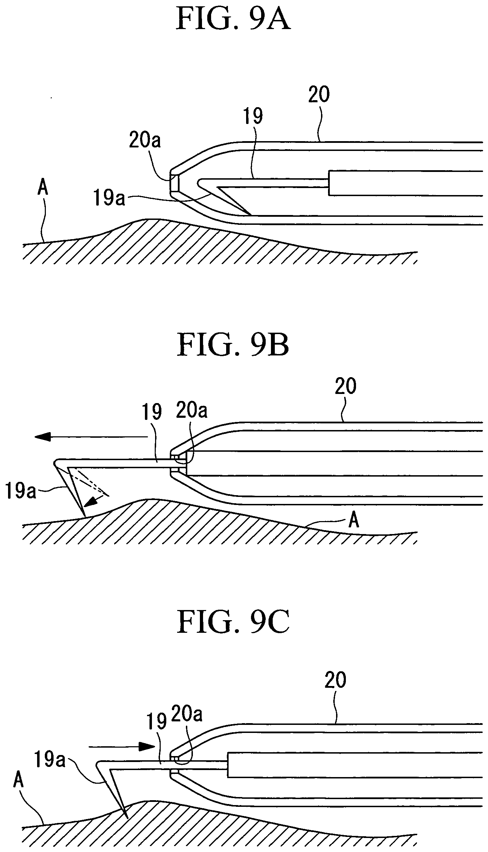

Furthermore, although the embodiment described above is an example provided with the attachment pad 7 pivotally joined by the joint 8 having the shaft 8a, alternatively, as shown in Figs. 9A to 9C, a hook member 19 that can be hooked to the heart A may be employed as the securing means 3. In the example shown in Figs. 9A to 9C, for example, the hook member 19 accommodated inside a cylindrical case member 20 is inserted into the body through a forceps channel (not shown) provided along the lengthwise direction of the inserted portion 2.

That is, inside the body, the hook member 19 folded and accommodated in the cylindrical case member 20 as shown in Fig. 9A is projected through a distal-end opening 20a of the cylindrical case member 20, whereby, as shown in Fig. 9B, a hook 19a at the distal end is opened so that the hook 19a readily hooks, and as shown in Fig. 9C, by slightly retracting the hook member 19, it is possible to hook the hook member 19 to tissue, such as the heart A. Accordingly, without supplying power, such as a negative pressure, it is possible to secure the distal end of the inserted portion 2 to tissue, such as the heart A. The number of hook members 19 is not limited to one, and a plurality of hook members 19 may be projected and hooked.

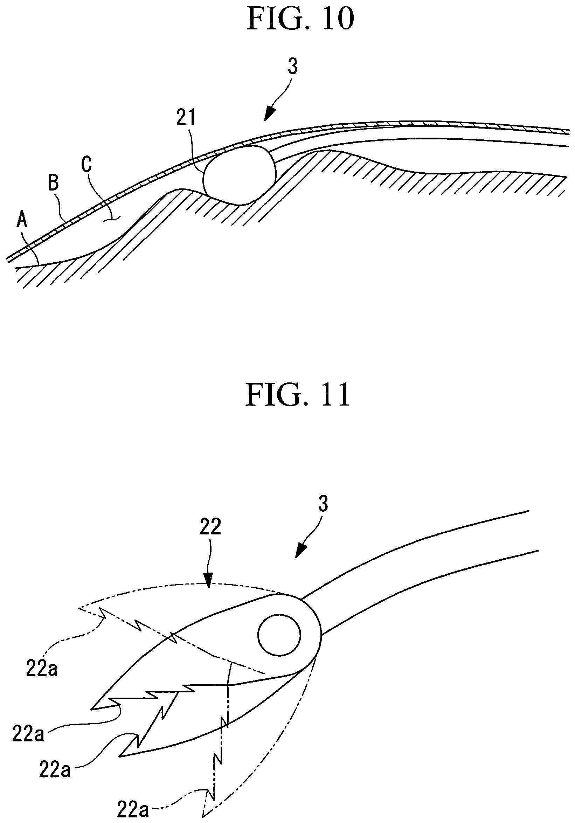

Alternatively, in a case where the tissue has projections and recesses, for example, in a case where it is possible to exploit projections and recesses on the surface of the heart A, a balloon member 21 may be employed as the securing means 3, as shown in Fig. 10. That is, similarly to the case described above, by projecting the balloon member 21 from the distal end of the inserted portion 2 via the forceps channel and inflating the balloon member 21 in the proximity of a recess on the surface of the heart A in the pericardial cavity C, it is possible to secure the inserted portion 2 held between the pericardium B and the surface of the heart A.

Instead of the balloon member 21, forceps 22 may be used as the securing means 3, as shown in Fig. 11. That is, by similarly projecting the forceps 22 from the distal end of the inserted portion 2 through the forceps channel and manipulating the forceps 22 to hold the surface of the heart A, it is possible to secure the inserted portion 2 to the surface of the heart A. The forceps 22 preferably have, at their holding regions, a plurality of projections 22a that can be engaged with tissue.

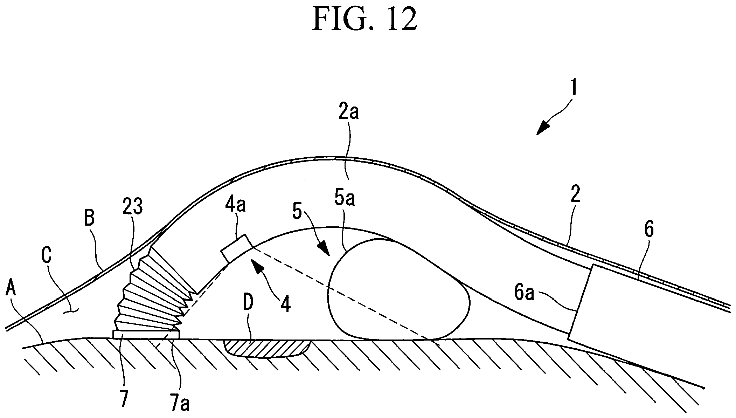

Furthermore, although the shaft 8a disposed along a radial direction of the inserted portion 2 is provided as the joint 8 that pivotally joins the attachment pad 7 at the distal end of the inserted portion 2 in this embodiment, alternatively, as shown in Fig. 12, the attachment pad 7 may be joined at the distal end of the inserted portion 2 by a tubular cylindrical member, such as bellows. Accordingly, by deforming the bellows 23, it is possible to arbitrarily change the angle of the attachment pad 7 relative to the inserted portion 2, and it is also possible to seal the space connected to the attachment pad 7 by the bellows 23 so that a negative pressure supplied to the attachment pad 7 is maintained.

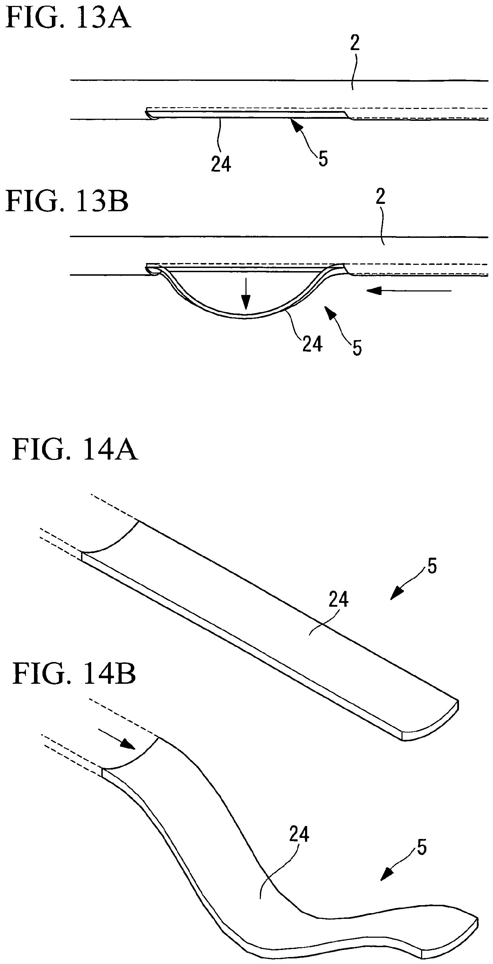

Furthermore, although the balloon 5a is employed as the observation-distance adjusting means 5 in this embodiment, alternatively, as shown in Figs. 13A and 13B and Figs. 14A and 14B, a strip-shaped elastic member 24 may be employed. For example, when the inserted portion 2 is inserted into the body, the elastic member 24 has a form extending on the outer surface of the inserted portion 2 along the lengthwise direction, as shown in Fig. 13A. The distal end of the elastic member 24 is fixed to the inserted portion 2, and the proximal end thereof is projected from the proximal end of the inserted portion 2 so that it can be pushed and pulled by an operator.

With the inserted portion 2 inserted into the body, when an operator pushes the proximal end of the elastic member 24 toward the distal end, the elastic member 24 curves and projects outward in a radial direction from the inserted portion 2, as shown in Fig. 13B. Thus, it is possible to push the surface of the heart A by the projected elastic member 24, and by adjusting the amount of projection, similarly to the case of the balloon 5a in the embodiment described above, it is possible to adjust the distance between the observation window 4a and the surface of the heart A.

As shown in Figs. 14A and 14B, the strip-shaped elastic member 24 has a wide shape extending over a predetermined range along the circumferential direction of the inserted portion 2, so that it is possible to stabilize the direction of projection in a radial direction.

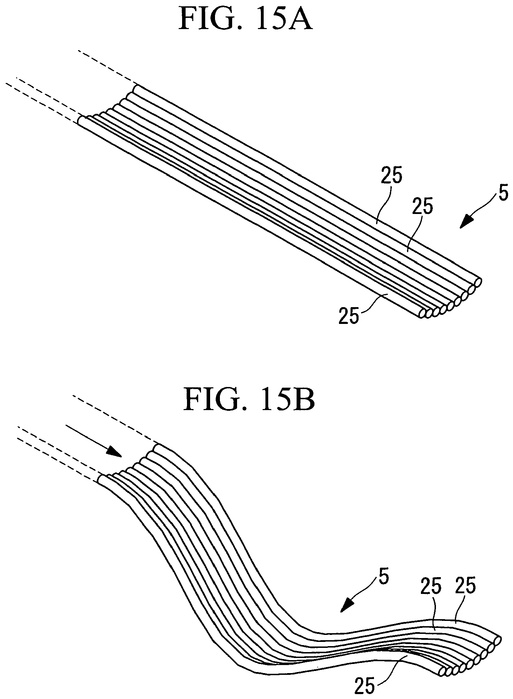

Instead of the strip-shaped elastic member 24, as shown in Figs. 15A and 15B, a plurality of wires 25 may be bundled such that they are arrayed along the circumferential direction of the inserted portion 2.

Accordingly, at the time of insertion into the body, as shown in Fig. 15A, a tension is applied, and after insertion into the body, as shown in Fig. 15B, the wires 25 are pressed toward the distal end and are thereby bent. Thus, it is possible to press the surface of the heart A and thereby adjust the distance between the observation window 4a and the surface of the heart A.

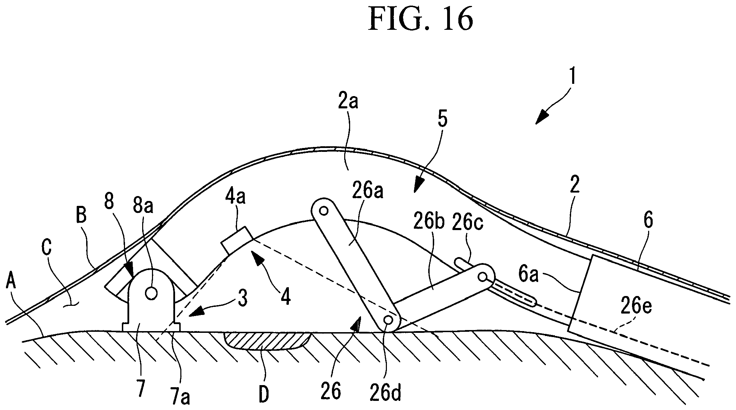

Alternatively, instead of the strip-shaped elastic member 24 or the plurality of wires 25 described above, as shown in Fig. 16, the observation-distance adjusting means 5 may be configured of a link mechanism 26 including a plurality of (two in Fig. 16) links 26a and 26b. In the example shown in Fig. 16, of the two links 26a and 26b pivotally coupled with each other, the distal end of the link 26a at the distal end is pivotally attached to the inserted portion 2, and by moving the proximal end of the link 26b at the proximal end along a guide groove 26c provided on the inserted portion 2, it is possible to project and retract a joint portion 26d between the links 26a and 26b in a radial direction.

For example, the link 26b at the proximal end is moved by an operation at the proximal end of the inserted portion 2 via a wire 26e.

Alternatively, as shown in Fig. 17B, the distal-end portion of the inserted portion 2 may be trained in advance into a predetermined curved shape, and at the time of insertion into the body, as shown in Fig. 17A, the inserted portion 2 may be accommodated in a guide sheath 27 that is capable of shaping the inserted portion 2 in an extended form. Accordingly, after the inserted portion 2 is inserted into the pericardial cavity C in the state accommodated in the guide sheath 27 as shown in Fig. 17A, by withdrawing the guide sheath 27 toward the proximal end as shown in Fig. 17B, the inserted portion 2 is released and is restored to the pretrained form. Thus, it is possible to readily adjust the distance between the observation window 4a and the surface of the heart A to a preset distance.

Furthermore, the inserted portion 2 may be provided with a plurality of magnets attached with spaces along the lengthwise direction thereof. Accordingly, even when the inserted portion 2 is inserted into the body of the patient, it is possible to identify the positions of the magnets by a magnetic sensor provided outside the body and to visualize the form of the inserted portion 2 in an image.

Furthermore, although the balloon 5a is disposed so as to project in one direction outside the circumference of the inserted portion 2 when the balloon 5a is used as the observation-distance adjusting means 5, alternatively, the balloon 5a may be inflated in all directions. Alternatively, the balloon 5a that can be inflated in all directions may be restrained by using a spool or the like so that it inflates only in one direction.

Furthermore, although the pericardial cavity C between the heart A and the pericardium B is described as an example of a region inside the body that is observed with the endoscope 1 in this embodiment, the observed region is not limited to the pericardial cavity C, and application to other arbitrary tissues is possible.

Next, an endoscope 30 according to an example useful for understanding the invention will be described below with reference to the drawings.

In the description of the endoscope 30 according to this example, parts that are configured the same as those of the endoscope 1 according to the first embodiment described above will be designated by the same reference signs, and descriptions thereof will be omitted.

As shown in Figs. 18 and 19, the endoscope 30 according to this example includes securing means 32 having suction holes 31 that are attached to the pericardium B instead of the securing means 3 having the attachment pad 7 that is attached to the surface of the heart A. As shown in Fig. 19, the suction holes 31 constituting the securing means 32 are provided on the side face of the inserted portion 2, and serve to attach the side face of the inserted portion 2 to the pericardium B by a negative pressure supplied via a duct (not shown).

Preferably, multiple suction holes 31 are provided, and the suction holes 31 are provided at intervals along the lengthwise direction of the inserted portion 2 and independently enable attachment with negative pressures. Accordingly, it is possible to switch the suction holes 31 used for attachment with negative pressures and thereby adjust the position where the inserted portion 2 is attached to the pericardium B.

Furthermore, in this example, an inserted portion 2 that itself is trained so as to curve when it is projected from the guide sheath 6, as shown in Fig. 18, is employed as the observation-distance adjusting means 5.

In the endoscope 30 according to this example, the observation optical system 4 that acquires an image at the front is provided at a distal-end face 2b of the inserted portion 2. Furthermore, a forceps channel (not shown) is formed to have an opening at the distal-end face 2b of the inserted portion 2 so that it is possible to project and retract an instrument for manipulating an affected area on the surface of the heart A.

With the thus-configured endoscope 30 according to this example, the inserted portion 2 is curved by projecting the inserted portion 2 of the endoscope 30 from inside the guide sheath 6 into the pericardial cavity C with the distal end of the guide sheath 6 inserted into the pericardial cavity C. Then, the inserted portion 2 is rotated about the lengthwise axis thereof, whereby the inserted portion 2 trained to curve widens the gap between the pericardium B and the surface of the heart A. That is, an observation space is provided in the pericardial cavity C between the pericardium B and the surface of the heart A. Accordingly, it is possible to direct the distal-end face 2b of the inserted portion 2 whose side face is attached to the pericardium B by the securing means 32 toward the surface of the heart A at a location separated from the surface of the heart A.

Compared with the pulsing heart A, the pericardium B can be considered as effectively stationary, and the inserted portion 2 attached to the pericardium B is held by the pericardium B so as not to be displaced considerably regardless of pulsation of the heart A. As a result, by the operation of the observation optical system 4 of the endoscope 30, it is possible to acquire sharp images over a relatively wide range on the surface of the pulsing heart A. That is, a doctor can endoscopically observe the surface of the heart A as if the doctor cut open the chest of the patient by a thoracotomy operation and cut the pericardium B to expose the surface of the heart A. This facilitates observation of a necrosed region of the heart A and a procedure on an affected area.

Although the securing means 32 having the one or more suction holes 31 formed on the side face of the inserted portion 2 is employed for securing the inserted portion 2 to the pericardium B in this example, alternatively, securing means having a single suction hole 31 may be employed. Alternatively, securing means may be employed in which negative pressures are supplied simultaneously to the plurality of suction holes 31. Furthermore, securing means may be employed in which a plurality of suction holes 31 is provided along the circumferential direction.

Instead of the securing means 32 having the suction holes 31, as shown in Fig. 20, it is possible to employ securing means 33 that is implemented by a holding part provided projectably and retractably on the side face of the inserted portion 2 and projected from the side face of the inserted portion 2 to hold the pericardium B disposed in proximity to the side face of the inserted portion 2. Alternatively, instead of the securing means 33 implemented by the holding part, as shown in Fig. 21, securing means 34 implemented by a hook that pierces the pericardium B may be employed.

Alternatively, instead of the securing means 32 having the suction holes 31, as shown in Fig. 22, it is possible to employ securing means 36 in which a jet 35 for jetting a fluid, for example, air, is provided on the side face of the inserted portion 2, and the inserted portion 2 is pressed against and secured to the pericardium B by the power of the fluid F jetted from the jet 35. In this case, just jetting the fluid F from the jet 35 would fill the inside of the pericardium B with the fluid F. Thus, a discharge duct 37 should be provided to have an opening on the pericardial cavity C so that the supplied fluid F will be discharged outside through the guide sheath 6.

Furthermore, in this case, since the inserted portion 2 is pressed against the pericardium B by the power of the fluid F jetting against the surface of the heart A, preferably, the flow level of the air jetted from the jet 35 is adjusted in synchronization with pulsation of the heart A so that the inserted portion 2 will not be affected by pulsation when the heart A pulses. That is, by adjusting the flow level of the air so that the amount is smaller when the heart A expands and the amount is greater when the heart A contracts, it is possible to minimize the effect of pulsation.

Alternatively, instead of the securing means 32 having the suction holes 31, as shown in Fig. 23 or Fig. 24, securing means implemented by a magnet (magnetic-force generating means) 38 disposed on the side face of the inserted portion 2 may be employed. In the case shown in Fig. 23, a magnet (magnetic-force generating means) 39 is fixed at the pericardium B, so that it is possible to attach the inserted portion 2 to the pericardium B by a magnetic attracting force between the magnet 39 at the pericardium B and the magnet 38 at the inserted portion 2.

On the other hand, in the case shown in Fig. 24, a magnet 40 (magnetic-force generating means) 40 is fixed inside the heart A, so that it is possible to press the inserted portion 2 toward and secure the inserted portion 2 to the pericardium B by a magnetic repelling force between the magnet 40 at the heart A and the magnet 38 at the inserted portion 2.

In this case, by using an electromagnet as at least one of the magnet 40 fixed inside the heart A and the magnet 38 provided at the inserted portion 2 and adjusting the magnetic force generated by the electromagnet, it is possible to adjust the magnetic repelling force. Observation-distance adjusting means may be implemented in this manner.

Furthermore, although the inserted portion 2 which itself is trained to curve is employed as the observation-distance adjusting means 5, alternatively, as shown in Fig. 25, a balloon 41 that is disposed between the inserted portion 2 and the surface of the heart A and that is inflated or deflated may be employed. By inflating the balloon 41 with the inserted portion 2 attached to the pericardium B, it is possible to form an observation space in the pericardial cavity C between the pericardium B and the surface of the heart A. Furthermore, it is possible to adjust the observation distance by changing the degree of inflation of the balloon 41. Furthermore, by pressing the balloon 41 against the surface of the heart A, it is possible to alleviate the pulsation of the heart A, thereby further suppressing blurring of images acquired by the observation optical system 4.

Furthermore, the endoscopes 1 and 30 according to the embodiments described above may be modified in the following manners.

First, a plurality of balloons 5a or 41 may be arrayed along the lengthwise direction of the inserted portion 2, which may be capable of being inflated or deflated independently.

For example, as shown in Fig. 26, from the guide sheath 6 inserted from the bottom of the xiphoid process E through the abdomen into the pericardial cavity C in the proximity of the heart apex H, the inserted portion 2 is inserted into the pericardial cavity C and curved into a substantially U shape at the pericardial turnover region J so that the distal-end face 2b is directed toward the heart apex H. In this state, as shown in Fig. 27, the balloons 41 are inflated, whereby an observation space indicated as a hatched region K is provided in the vicinity of the distal-end face 2b of the inserted portion 2 in the pericardial cavity C between the surface of the heart A and the pericardium B. Furthermore, as shown in Fig. 28, the inserted portion 2 is secured in a state pressed against the pericardium B by the balloons 41.

Furthermore, by curving the curving portion 2a of the inserted portion 2 in this state, a wide field of view for the observation optical system 4 is provided in the space surrounded by the balloons 41 and formed between the pericardium B and the surface of the heart A, so that it is possible to perform detailed observation. Furthermore, by adjusting the amount of inflation of the balloons 41 in synchronization with pulsation timing, it is possible to further stabilize the field of view. That is, by adjusting the amount of inflation of the balloons 41 so that it becomes smaller when the heart A expands and it becomes greater when the heart A contracts, it is possible to maintain the distance between the pericardium B and the surface of the heart A constant, so that the field of view is further stabilized.

In this case, as shown in Fig. 29, it is possible to ensure only the minimum necessary space by selecting the balloons 41 to be inflated as needed.

Secondly, instead of the balloons 41, as shown in Fig. 30 or Fig. 31, observation-distance adjusting means may be implemented by a spring 42 or a retention bar 43 that is released and expanded when the inserted portion 2 is projected from the guide sheath 6. Preferably, the retention bar 43 has such a structure that it projects so as to come in contact with the surface of the heart A at two or more positions. Accordingly, it is possible to stabilize the inserted portion 2 so that it is not twisted by pulsation of the heart A.

Alternatively, the retention bar 43 may be projected from the guide sheath 6, as shown in Fig. 33.

Alternatively, a retention bar may be configured of a link mechanism 26 similar to that shown in Fig. 16, and the amount of projection may be adjusted by the link mechanism 26.

Alternatively, a space may be provided in the pericardial cavity C between the pericardium B and the surface of the heart A by using a basket 44 formed of wires and configured to expand when the inserted portion 2 is projected from the guide sheath 6, as shown in Fig. 34, or by using tubular members 45 or a wire member trained to curve as shown in Fig. 35.

Alternatively, in order to stabilize the inserted portion 2 projected from the guide sheath 6 against pulsation of the heart A, as shown in Fig. 36, a stabilizer may be provided that is formed of wires 46 projecting in radial directions from either side face of the inserted portion 2 and coming into contact with the surface of the heart A.

Alternatively, suction holes 47 may be provided that serve to attach the inserted portion 2 to the surface of the heart A in the proximity of the opening 6a of the guide sheath 6. A plurality of suction holes 47 may be provided with spaces along the lengthwise axis of the inserted portion 2, as shown in Fig. 37, or may be provided along the circumferential direction, as shown in Fig. 38.

Alternatively, as shown in Figs. 39A and 39B, an inserted portion 2 may be employed that itself has a flatly expanding structure. Fig. 39A shows the inserted portion 2 tightly bundled so that it can pass through the guide sheath 6, and Fig. 39B shows the inserted portion 2 flatly expanded after it is projected from the opening 6a of the guide sheath 6.

Alternatively, an inserted portion 2 having a flat sectional shape may be employed, as shown in Fig. 40.

In the figure, reference sign 48 denotes an illumination light source.

Alternatively, as shown in Fig. 41, an inserted portion 2 may be employed that has a flat shape in which a plurality of tubular members having circular sections are arrayed in one line and bundled. In this case, preferably, the tubular members 49 include a tubular member having the observation optical system 4 and tubular members having channel exits 10. {0088}

Alternatively, as shown in Fig. 42, balloons 50 may be inflated from two circumferentially deviated positions of the inserted portion 2. Accordingly, since the surface of the heart A is pressed simultaneously by the balloons 50 at the two positions, it is possible to stably support the inserted portion 2 while suppressing pulsation of the heart A.

Alternatively, as shown in Fig. 43, the inserted portion 2 may be stabilized by employing a balloon 51 that expands into a shape having an elongated circular section.

The embodiments have been described above in the context of examples where an endoscope is inserted between the heart and the pericardium to observe the heart. Alternatively, it is possible to insert an endoscope between other pairs of organs, for example, the lungs, the stomach, the gallbladder, the pancreas, the spleen, the intestines, the kidneys, the urinary bladder, the uterus, the peritoneum, the pleura, the thoracic diaphragm, between brain tissues, or between skeletal muscles to observe these organs. When an endoscope is inserted into a gap between mutually adjacent organs, since the endoscope is pressed by the organs on either side, it is difficult to provide a sufficient observation distance between the observation window and the organ surfaces. Particularly, when these organs adhere to each other, it is even more difficult to provide an observation distance. However, with an endoscope according to the present invention, even in the case of adhering organs, it is possible to provide an appropriate observation distance by a simple operation, allowing the surfaces of the organs to be readily observed.

### {Reference Signs List}

- A:: heart (tissue)
- 1:: endoscope
- 2:: inserted portion
- 2a:: curving portion
- 3:: securing means
- 4:: observation optical system
- 5:: observation-distance adjusting means
- 5a:: balloon
- 7:: attachment pad (attachment unit)
- 7a:: attachment surface
- 7b:: suction hole
- 8:: joint
- 8a:: shaft (axis)
- 10:: exit
- 11:: instrument
- 19:: hook member
- 21:: balloon member
- 22:: forceps
- 23:: bellows (tubular member)
- 24:: elastic member
- 25:: wire
- 26:: link mechanism
- 26a, 26b:: link (link member)
- 26d:: joint portion
- 27:: guide sheath

## Claims

1. An endoscope (1) comprising:
an inserted portion (2) that is adapted to be inserted into the body of a patient;
securing means (3) for securing the inserted portion (2) to tissue inside the body, the securing means (3) being provided at least at a distal-end portion of the inserted portion (2);
an observation optical system (4) that is provided at the inserted portion (2) and that is adapted to acquire an image of the tissue or adjacent tissue adjacent to the tissue; and
observation-distance adjusting means (5) for adjusting the distance between the observation optical system (4) and a surface of the tissue or a surface of the adjacent tissue,
wherein the observation optical system (4) is provided at a different position from a position where the securing means (3) is provided along a lengthwise direction,
wherein the observation-distance adjusting means (5) is provided at a different position from the position where the securing means (3) is provided along the lengthwise direction, and **characterized in that** the securing means includes an attachment unit (7, 8) that is adapted to attach to the tissue by using a negative pressure, and
the attachment unit (7, 8) has an attachment surface (7a) having a plurality of suction holes (7b) for attachment to the tissue and is configured such that the angle of the attachment surface (7a) relative to the inserted portion (2) is changeable, wherein the negative pressure is independently provided to the plurality of suction holes (7b), so that the suction holes (7b) can be attached to the tissue individually.

2. An endoscope according to Claim 1, wherein the attachment unit (7, 8) includes an attachment pad (7) having the attachment surface (7a) and a joint (8) that joins the attachment pad (7) with the inserted portion (2) so that the attachment pad (7) is pivotable about an axis perpendicular to a lengthwise axis of the inserted portion (2).

3. An endoscope according to Claim 2, wherein the joint is a flexible tubular member (23) that is adapted to convey a negative pressure to the attachment pad (7).

4. An endoscope according to Claim 3, wherein the tubular member (23) is a bellows.

5. An endoscope according to any one of Claims 1 to 4, wherein the observation-distance adjusting means is implemented by a balloon (5a) that is adapted to be disposed between the tissue or the adjacent tissue and the inserted portion (2) and that is adapted to be inflated or deflated to adjust the distance between the observation optical system (4) and a surface of the tissue or a surface of the adjacent tissue.

6. An endoscope according to Claim 1, wherein the observation-distance adjusting means is implemented by training the inserted portion (2) into a curved shape.

7. An endoscope according to Claim 6, wherein the observation-distance adjusting means includes a guide sheath (6) that is adapted to accommodate the inserted portion (2) in an extended state such that the inserted portion (2) is projectable and retractable from a distal end (6a) of the guide sheath (6).

8. An endoscope according to any one of Claims 1 to 7, wherein the observation-distance adjusting means is implemented by an elastic member (24) that is disposed on a side face of the inserted portion along the lengthwise direction, that is fixed at a distal end of the inserted portion (2), and that is adapted to be projected in a radial direction by being pushed in the lengthwise direction from a proximal end of the inserted portion (2).

9. An endoscope according to Claim 8, wherein the elastic member has a cross-sectional shape extending in a circumferential direction of the inserted portion (2).

10. An endoscope according to Claim 8, wherein the elastic member is formed of a bundle of wires (25) arrayed in the circumferential direction of the inserted portion (2).

11. An endoscope according to any one of Claims 1 to 10, wherein the observation-distance adjusting means is a link mechanism (26) that includes a plurality of link members (26a, 26b) coupled so as to pivot with each other and that is adapted to move a joint portion (26d) in a radial direction when one end thereof is moved in the lengthwise direction of the inserted portion (2).

12. An endoscope according to any one of Claims 1 to 11, wherein the inserted portion (2) has a channel for passing various instruments (11) in accordance with procedures to be performed on the tissue or the adjacent tissue.

13. An endoscope according to Claim 12,
wherein the observation optical system (4) is provided on a side face of the inserted portion (2), and
wherein the channel has an exit (10) provided at a circumferential position substantially corresponding to the observation optical system (4).

14. An endoscope according to Claim 12, wherein the channel has an exit (10) provided so as to permit an instrument (11) projected from the exit to pass through a field of view of the observation optical system (4).

## Patentansprüche

1. Endoskop (1), umfassend:
einen eingeführten Abschnitt (2), der dazu angepasst ist, in den Körper eines Patienten eingeführt zu werden;
Befestigungsmittel (3) zum Befestigen des eingeführten Abschnitts (2) am Gewebe im Innern des Körpers, wobei das Befestigungsmittel (3) mindestens an einem Distalendabschnitt des eingeführten Abschnitts (2) vorgesehen ist;
ein optisches Beobachtungssystem (4), das an dem eingeführten Abschnitt (2) vorgesehen und dazu angepasst ist, ein Bild von dem Gewebe oder von an das Gewebe angrenzendem Gewebe aufzunehmen; und
Beobachtungsabstandseinstellmittel (5) zum Einstellen des Abstands zwischen dem optischen Beobachtungssystem (4) und einer Oberfläche des Gewebes oder einer Oberfläche des angrenzenden Gewebes,
wobei das optische Beobachtungssystem (4) an einer Position vorgesehen ist, die von der Position verschieden ist, an der das Befestigungsmittel (3) entlang einer Längsrichtung vorgesehen ist,
wobei das Beobachtungsabstandseinstellmittel (5) an einer Position vorgesehen ist, die von der Position verschieden ist, an der das Befestigungsmittel (3) entlang einer Längsrichtung vorgesehen ist, und
**dadurch gekennzeichnet, dass**
das Befestigungsmittel eine Anhafteinheit (7, 8) umfasst, die dazu angepasst ist, mithilfe eines Unterdrucks an dem Gewebe anzuhaften, und
die Anhafteinheit (7, 8) eine Anhaftfläche (7a) aufweist, die eine Mehrzahl von Sauglöchern (7b) zur Anhaftung an dem Gewebe aufweist und derart ausgelegt ist, dass der Winkel der Anhaftfläche (7a) bezogen auf den eingeführten Abschnitt (2) verändert werden kann,
wobei der Unterdruck der Mehrzahl von Sauglöchern (7b) unabhängig zur Verfügung gestellt wird, sodass die Sauglöcher (7b) individuell an dem Gewebe angehaftet werden können.

2. Endoskop nach Anspruch 1, wobei die Anhafteinheit (7, 8) ein Anhaftpolster (7) umfasst, das die Anhaftfläche (7a) und ein Gelenk (8) aufweist, das das Anhaftpolster (7) derart mit dem eingeführten Abschnitt (2) verbindet, dass das Anhaftpolster (7) um eine Achse senkrecht zu einer Längsachse des eingeführten Abschnitts (2) gedreht werden kann.

3. Endoskop nach Anspruch 2, wobei das Gelenk ein flexibles röhrenförmiges Element (23) ist, das dazu angepasst ist, einen Unterdruck auf das Anhaftpolster (7) zu übertragen.

4. Endoskop nach Anspruch 3, wobei das röhrenförmige Element (23) ein Faltenbalg ist.

5. Endoskop nach einem der Ansprüche 1 bis 4, wobei das Beobachtungsabstandseinstellmittel durch einen Ballon (5a) implementiert wird, der dazu angepasst ist, zwischen dem Gewebe oder dem angrenzenden Gewebe und dem eingeführten Abschnitt (2) angeordnet zu sein, und der dazu angepasst ist, aufgeblasen oder entleert zu werden, um den Abstand zwischen dem optischen Beobachtungssystem (4) und einer Oberfläche des Gewebes oder einer Oberfläche des angrenzenden Gewebes einzustellen.

6. Endoskop nach Anspruch 1, wobei das Beobachtungsabstandseinstellmittel durch Ausbilden des eingeführten Abschnitts (2) in eine gekrümmte Form implementiert wird.

7. Endoskop nach Anspruch 6, wobei das Beobachtungsabstandseinstellmittel eine Führungshülle (6) umfasst, die dazu angepasst ist, den eingeführten Abschnitt (2) in einem ausgefahrenen Zustand unterzubringen, sodass der eingeführte Abschnitt (2) von einem distalen Ende (6) der Führungshülle (6) ausgefahren und zurückgezogen werden kann.

8. Endoskop nach einem der Ansprüche 1 bis 7, wobei das Beobachtungsabstandseinstellmittel durch ein elastisches Element (24) implementiert wird, das an einer Seitenfläche des eingeführten Abschnitts entlang der Längsrichtung angeordnet ist, das an einem distalen Ende des eingeführten Abschnitts (2) befestigt ist, und das dazu angepasst ist, in einer Radialrichtung vorzuragen, indem es in der Längsrichtung von einem proximalen Ende des eingeführten Abschnitts (2) gedrückt wird.

9. Endoskop nach Anspruch 8, wobei das elastische Element eine Querschnittsform aufweist, die sich in eine Umfangsrichtung des eingeführten Abschnitts (2) erstreckt.

10. Endoskop nach Anspruch 8, wobei das elastische Element aus einem Bündel Kabel (25) gebildet ist, die in der Umfangsrichtung des eingeführten Abschnitts (2) angeordnet sind.

11. Endoskop nach einem der Ansprüche 1 bis 10, wobei das Beobachtungsabstandseinstellmittel ein Verbindungsmechanismus (26) ist, der eine Mehrzahl Verbindungselemente (26a, 26b) umfasst, die derart gekoppelt sind, dass sie miteinander drehen, und der dazu angepasst ist, einen Gelenkabschnitt (26d) in eine Radialrichtung zu bewegen, wenn ein Ende davon in die Längsrichtung des eingeführten Abschnitts (2) bewegt wird.

12. Endoskop nach einem der Ansprüche 1 bis 11, wobei der eingeführte Abschnitt (2) einen Kanal zum Durchführen verschiedener Instrumente (11) gemäß den an dem Gewebe oder dem angrenzenden Gewebe durchzuführenden Prozeduren aufweist.

13. Endoskop nach Anspruch 12,
wobei das optische Beobachtungssystem (4) an einer Seitenfläche des eingeführten Abschnitts (2) vorgesehen ist, und
wobei der Kanal einen Ausgang (10) aufweist, der an einer Umfangsposition vorgesehen ist, die im Wesentlichen dem optischen Beobachtungssystem (4) entspricht.

14. Endoskop nach Anspruch 12, wobei der Kanal einen Ausgang (10) aufweist, der derart vorgesehen ist, dass er zulässt, dass ein Instrument (11), das von dem Ausgang vorragt, durch ein Sichtfeld des optischen Beobachtungssystems (4) durchgeht.

## Revendications

1. Endoscope (1) comprenant :
une partie insérée (2) qui est conçue pour être insérée dans le corps d'un patient ;
des moyens de fixation (3) pour fixer la partie insérée (2) à un tissu à l'intérieur du corps, les moyens de fixation (3) étant disposés au moins à une partie d'extrémité distale de la partie insérée (2) ;
un système optique d'observation (4) qui est disposé à la partie insérée (2) et qui est conçu pour acquérir une image du tissu ou d'un tissu adjacent qui est adjacent au tissu ; et
des moyens d'ajustement de distance d'observation (5) pour ajuster la distance entre le système optique d'observation (4) et une surface du tissu ou une surface du tissu adjacent,
le système optique d'observation (4) étant disposé dans une position différente d'une position dans laquelle les moyens de fixation (3) sont disposés le long d'une direction longitudinale,
les moyens d'ajustement de distance d'observation (5) étant disposés dans une position différente de la position dans laquelle les moyens de fixation (3) sont disposés le long de la direction longitudinale, et
**caractérisé par le fait que**
les moyens de fixation comprennent une unité d'attache (7, 8) qui est conçu pour s'attacher au tissu en utilisant une pression négative, et
l'unité d'attache (7, 8) a une surface d'attache (7a) ayant une pluralité de trous d'aspiration (7b) en vue d'une attache au tissu, et est configurée de telle sorte que l'angle de la surface d'attache (7a) par rapport à la partie insérée (2) peut être changé,
la pression négative étant fournie de manière indépendante à la pluralité de trous d'aspiration (7b), de telle sorte que les trous d'aspiration (7b) peuvent être attachés individuellement au tissu.

2. Endoscope selon la revendication 1, dans lequel l'unité d'attache (7, 8) comprend un coussinet d'attache (7) ayant la surface d'attache (7a) et un joint (8) qui relie le coussinet d'attache (7) à la partie insérée (2) de telle sorte que le coussinet d'attache (7) est apte à pivoter autour d'un axe perpendiculaire à un axe longitudinal de la partie insérée (2).

3. Endoscope selon la revendication 2, dans lequel le joint est un élément tubulaire souple (23) qui est conçu pour transférer une pression négative au coussinet d'attache (7).

4. Endoscope selon la revendication 3, dans lequel l'élément tubulaire (23) est un soufflet.

5. Endoscope selon l'une quelconque des revendications 1 à 4, dans lequel les moyens d'ajustement de distance d'observation sont mis en oeuvre par un ballonnet (5a) qui est conçu pour être disposé entre le tissu ou le tissu adjacent et la partie insérée (2) et qui est conçu pour être gonflé ou dégonflé afin d'ajuster la distance entre le système optique d'observation (4) et une surface du tissu ou une surface du tissu adjacent.

6. Endoscope selon la revendication 1, dans lequel les moyens d'ajustement de distance d'observation sont mis en oeuvre en entraînant la partie insérée (2) en une forme incurvée.

7. Endoscope selon la revendication 6, dans lequel les moyens d'ajustement de distance d'observation comprennent une gaine de guidage (6) qui est conçue pour recevoir la partie insérée (2) dans un état étendu, de telle sorte que la partie insérée (2) peut faire saillie et se rétracter depuis une extrémité distale (6a) de la gaine de guidage (6).

8. Endoscope selon l'une quelconque des revendications 1 à 7, dans lequel les moyens d'ajustement de distance d'observation sont mis en oeuvre par un élément élastique (24) qui est disposé sur une face latérale de la partie insérée le long de la direction longitudinale, qui est fixé au niveau d'une extrémité distale de la partie insérée (2), et qui est conçu pour faire saillie dans une direction radiale en étant poussé dans la direction longitudinale depuis une extrémité proximale de la partie insérée (2).

9. Endoscope selon la revendication 8, dans lequel l'élément élastique a une forme en section transversale s'étendant dans une direction circonférentielle de la partie insérée (2).

10. Endoscope selon la revendication 8, dans lequel l'élément élastique est formé d'un faisceau de fils (25) disposés en réseau dans la direction circonférentielle de la partie insérée (2).

11. Endoscope selon l'une quelconque des revendications 1 à 10, dans lequel les moyens d'ajustement de distance d'observation sont un mécanisme de liaison (26) qui comprend une pluralité d'éléments de liaison (26a, 26b) couplés de façon à pivoter les uns avec les autres, et qui est conçu pour déplacer une partie de joint (26d) dans une direction radiale lorsqu'une extrémité de celle-ci est déplacée dans la direction longitudinale de la partie insérée (2).

12. Endoscope selon l'une quelconque des revendications 1 à 11, dans lequel la partie insérée (2) a un canal pour faire passer divers instruments (11) conformément aux procédures à réaliser sur le tissu ou le tissu adjacent.

13. Endoscope selon la revendication 12, dans lequel
le système optique d'observation (4) est disposé sur une face latérale de la partie insérée (2), et
le canal a une sortie (10) prévue dans une position circonférentielle correspondant sensiblement au système optique d'observation (4).

14. Endoscope selon la revendication 12, dans lequel le canal a une sortie (10) prévue de façon à permettre à un instrument (11), faisant saillie depuis la sortie, de traverser un champ de vision du système optique d'observation (4).
